# EUROPEAN PATENT SPECIFICATION

(11) **EP 0 327 312 B1**
(45) Date of publication and mention of the grant of the patent: **06.04.1994**
(21) Application number: 89300928.2
(22) Date of filing: 31.01.1989
(51) Int. Cl.: G01N 33/58, G01N 33/535, G01N 33/94, G01N 33/78

(54) **Receptor preincubation enzyme assay**
Rezeptorpreincubations-Enzymtest
Essai enzymatique de récepteur par préincubation

(30) Priority: 02.02.1988 US 151412
(43) Date of publication of application: 09.08.1989
(73) Proprietor: MICROGENICS CORPORATION (a Delaware corporation), Concord California 94520 (US)
(72) Inventor: Khanna, Pyare L., Fremont, CA 94539 (US); Friedman, Stephen B., Chapel Hill, NC 27516 (US); Dworschak, Robert T., Antioch, CA 94509 (US)
(74) Representative: Harrison, David Christopher

(56) References cited:
- EP-A- 0 055 869
- US-A- 4 708 929

## Description

The present invention relates to enzyme immunoassays and, in particular, to β-galactosidase complementation assays.

A wide variety of immunoassays have been based on the ability of fragments of β-galactosidase to complement and form active enzyme. In particular, a β-galactosidase enzyme donor (ED) combines with a β-galactosidase enzyme acceptor (EA) to form active β-galactosidase enzyme. Conjugating a small analyte or an analyte analogue to the ED at certain sites does not affect the ability to form β-galactosidase by a complementation reaction and hence rate of β-galactosidase catalyzed activity. However, when the ED-analyte conjugate is bound by anti-analyte antibody, the complementation rate is impeded, thereby the enzyme-catalyzed reaction rate during the initial phase of the reaction is reduced. This reduction in enzyme-catalyzed reaction rate has been used to quantitate the determination of a plurality of analytes on a principle of competitive inhibition where ED-analyte conjugate present in a reaction mixture and analyte present in the sample compete for anti-analyte antibody prior to the addition of EA. The polymerization rate of β-galactosidase formation and hence enzyme catalyzed reaction rate is increased as the amount of analyte present in the sample increased.

### Relevant Literature

Modified β-galactosidase enzyme donors and enzyme acceptors have been prepared by chemical synthesis and recombinant engineering. The modified fragments retain β-galactosidase activity upon complementation and facilitate production of and attachment of analyte to the fragments. See for example U.S. Patent No. 4,708,929 which discloses an assay using a β-galactosidase enzyme acceptors and donor system in a sequential additional assay.

### SUMMARY OF THE INVENTION

A β-galactosidase complementation immuno assay method for determining the concentration of an analyte in a sample is provided as set out in claim 4 and claim 2. The method is based on comgate/anti-analyte antibody complex with analyte present in the sample. The method comprises reacting a β-galactosidase ED-analyte conjugate/anti-analyte antibody complex, a β-galactosidase enzyme acceptor (EA), enzyme substrate and sample and determining the rate of enzyme-catalyzed reaction as indicative of the amount of analyte present in the sample. Usually, the EA is added to the reaction mixture after a time for the anti-analyte antibody of the complex to react with analyte present in the sample.

### DESCRIPTION OF THE SPECIFIC EMBODIMENTS

A β-galactosidase complementation assay method for determining the concentration of an analyte is provided. The method utilizes a preformed ED-analyte conjugate/anti-analyte antibody complex. The complex is combined with analyte present in the sample for a time period sufficient for anti-analyte antibody present in the complex to react with analyte present in the sample. EA and enzyme substrate are added to form a reaction mixture having β-galactosidase activity. Conveniently, EA is added to the combination of ED-analyte conjugate/anti-analyte antibody complex and sample after a period of time for the reaction of the complex with analyte. The rate of β-galactosidase-catalyzed substrate breakdown is determined in a preselected time period as indicative of the amount of analyte present in the sample.

The assay method can be used to quantitate any analyte determined by prior art immunoassay or complementation methods. The assay method can be used with any aqueous sample containing the analyte. The analyte may be an immunogen, e.g. a peptide, protein or carbohydrate having a high molecular weight (MW >1000), but usually will be a hapten (MW <1000) present in a bodily fluid, most usually in patient serum or plasma. Other than the removal of particulates, no pretreatment of the sample will usually be performed for purposes of the instant assay method.

The enzyme donor and enzyme acceptor are partial sequences of β-galactosidase, the enzyme donor being smaller. Either monomer may be mutated. The β-galactosidase enzyme donor fragment has substantially the same amino acid sequence as the N-terminal portion of the native β-galactosidase monomer. The enzyme acceptor has substantially the same or the same sequence as the C-terminus, being more than 50% of the β-galactosidase monomer molecule. β-Galactosidase activity results from complementation of the enzyme donor conjugate with the β-galactosidase enzyme acceptor fragment.

The enzyme acceptor is characterized by providing in conjunction with the enzyme donor conjugate a complex whose enzyme activity varies in relation to the amount of analyte present in the reaction mixture under the conditions of the assay. That is, when the enzyme donor-analyte conjugate/anti-analyte antibody complex reacts with analyte present in the sample, the enzyme activity during the time of measurement is substantially increased in comparison to the enzyme activity when no analyte is added to the reaction mixture.

β-Galactosidase enzyme donors and acceptors are described in U.S. Patent No. 4,708,929. The conditions of the assay described in that application are applicable to the subject invention.

The anti-analyte antibody may be either polyclonal or monoclonal. Preparation of anti-analyte antibodies is well known and constitutes no part of the present invention. Numerous polyclonal and monoclonal antibodies are available commercially. Alternatively, a receptor which specifically binds an analyte can be used in place of the antibody, e.g. vitamin B12-intrinsic factor and folic acid-folate binding protein.

The assay conditions and the reaction buffer used provide for complementation between enzyme donor and enzyme acceptor. In general, physiological buffers such as phosphate buffered saline, tris buffer and like buffers are useful. A preferred buffer comprises about 100 mM to about 300 mM NaPO₄, about 5 mM to about 10 mM EGTA, and about 10 mM to 20 mM NaN₃ having a pH of between 6 and 8. The temperature will usually be at least about 25°C, preferably elevated, but below 60°C. Enzyme assays are generally conducted at between room temperature (25°C) to less than about 40°C, most usually about 37°C. Optimal results were achieved at about 37°C. The assays are performed at atmospheric pressure.

The concentration of enzyme donor conjugates in the assay medium will usually be in the range of about 1 nM to about 60 nM, more usually about 5 nM to about 25 nM. The enzyme acceptor will usually be in substantial excess. The molar ratios of enzyme donor conjugate to enzyme acceptor will usually be 1:30 to 1:200, usually 1:50 to 1:150. The concentration of the enzyme donor-analyte conjugate will exceed the highest concentration of the analyte anticipated to be encountered in the sample.

As a first step for determining the amount of analyte present in a sample, a β-galactosidase ED-analyte conjugate/anti-analyte antibody complex and the sample are incubated for a time sufficient for the complex to react with analyte present in the sample to form a first reaction mixture. The ED-analyte conjugate/anti-analyte antibody complex is conveniently formed in a preincubation step by combining ED-analyte conjugate with anti-analyte antibody in an aqueous medium for a time sufficient for substantially complete reaction. A complete reaction of ED-analyte with anti-analyte antibody usually takes at least about 30 min. at 37°C. Thereafter, the complex may be used in an assay or stored in liquid or, conveniently, dry form for later use.

The ED-analyte and anti-analyte antibody are present in amounts so that substantially all ED present in the reaction mixture is bound to anti-analyte antibody, and all anti-analyte antibody present in the reaction mixture is bound to ED-analyte conjugate. Therefore, the concentration of antibody will vary depending on the desired assay range and the class of antibody as well as the binding affinity for the analyte under the assay conditions. For example, the normal ratio of IgM to ED-analyte will be 1:10 in binding sites while the ratio of IgG to ED-analyte will be 1:2 in binding sites. To determine when the antibody and ED-analyte conjugate are present in appropriate ratios, a titration of antibody concentration is performed to determine the minimum concentration of antibody necessary to achieve enzyme shutdown.

By "enzyme shutdown" is meant the lowest enzyme activity possible in the presence of ED-analyte conjugate, anti-analyte antibody, EA and substrate. When the antibody binding site concentration is significantly less than the ED-analyte concentration, an increased enzyme-catalyzed background activity rate is observed. When sufficient antibody is present and ED-analyte conjugate is titrated appropriately, the enzyme rate reaches its lowest activity (shutdown). Thereafter, excess antibody produces no significant change in the rate of enzyme-catalyzed reaction, but also a small amount of analyte added to the reaction mixture does not modulate the inhibition of complementation and hence the enzyme-catalyzed reaction rate. Of course, the concentration of the antibody may vary widely depending on its affinity for the the analyte and the desired range of the assay. Therefore the ratio of the concentration of ED-analyte conjugate and anti-analyte antibody will be such as to substantially acheive shutdown. Usually the concentrations of antibody and conjugate will be within 85%, more usually within 95% of the concentration necessary to achieve shutdown. Additionally, adding a second antibody specific for the Fc portion of the anti-analyte antibody to the reaction mixture prior to addition of EA lowered the β-galactosidase-catalyzed reaction rate at shutdown when using polyclonal antisera. However, the use of a second antibody was not found to make a significant difference when using monoclonal antisera.

The incubation of the ED-analyte conjugate/anti-analyte antibody complex with sample to form a first reaction mixture is for a time sufficient for the complex to react with analyte. Usually the incubation is at about 37°C for at least about 5 min., more usually for about 20 to 50 min. or more. Usually, EA is not present in the reaction mixture as a longer reaction period is required for displacement of the ED-analyte conjugate by analyte present in the sample to occur. Presence of EA during displacement step will result in excessive background signal accumulation and hence difficulty in detecting a difference in the enzyme catalyzed-reaction rates in analyte-containing and analyte-free reaction mixtures.

When EA was added at the same time as a sample (i.e. a 0 min. incubation), no difference between 0% analyte and a high calibrator analyte control (designated "100% analyte control" in the Experimental section) rate was observed when measuring the difference in optical density at 7 and 5 min. (A "100% analyte control" or "high calibrator" as used herein is a control sample having an infinite concentration of analyte, i.e. an analyte concentration such that further addition of analyte does not affect the reaction rate.) Therefore, when EA is added to the reaction mixture at the same time as sample, a substantially longer period of time is required to observe the effect of analyte on the rate.

Conveniently, the complex is incubated with sample prior to the addition of EA. A difference in the reaction rate following additon of EA was observed after only 2 to 6 min. of incubation of complex with sample in an exemplary digoxin assay using polyclonal anti-digoxin antibodies. However, the difference in reaction rates between a high calibrator and a 0% analyte control will conveniently be at least about 25% when those rates are measured within a few minutes (usually, less than 10 min.) following addition of EA. To determine an optimal incubation time for that step under the particular assay conditions, that difference can be measured at a number of incubation times. A shorter incubation time for formation of the ED-analyte conjugate/anti-antibody complex may be required when using monoclonal antibodies.

Varying amounts of sample can be used. When the sample is serum, usually the sample will comprise at least about 10-50% of the volume of the ED-analyte/anti-analyte antibody/sample reaction mixture. However, a serum sample may constitute two to four times the volume of the first reaction mixture to form two-thirds to four-fifths of the second reaction mixture.

The amount of enzyme activity in the medium is determined as indicative of the amount of analyte present in the sample in accordance with standard techniques for measuring β-galactosidase activity. An enzyme substrate is employed that when cleaved by the enzyme results in a change of the amount of light absorbance (optical density) or emission of the assay medium. That is, cleavage of the substrate results in the appearance or disappearance of a colored or fluorescent product. A preferred enzyme substrate is chlorophenol red galactoside (CPRG). CPR (chlorophenol red, the product) absorbance values are measured at 574 nm. CPRG and other comparable enzyme substrates such as ortho-nitrophenyl-β-D-galactoside (ONPG) are commercially available. When using CPRG, the reaction mixture will conveniently be incubated for from 1 to 10 min. following addition of EA prior to a first determination of optical density. There will usually be 1 to 5, more usually 2 to 4 min. between the first optical density reading and the second reading. Enzyme substrate can be added to the reaction either with the preformed ED-analyte conjugate/anti-analyte antibody complex, sample or EA.

An exemplary method for determining the amount of digoxin present in a serum sample uses polyclonal anti-digoxin antibodies and second antibodies specific for the Fc portion of the anti-analyte antibodies. The method comprises the following steps in the following order: (a) About 30 mM to 40 mM β-galactosidase ED-digoxin conjugate is incubated in a reaction buffer with polyclonal anti-digoxin antibody and second antibody for about 10 to about 30 min. at about 37°C to form a first reaction mixture. The anti-digoxin antibody is present in the reaction mixture in a sufficient amount in comparison to ED-analyte conjugate to substantially achieve shutdown. (b) An equal volume of the sample is combined with the first reaction mixture to form a second reaction mixture. (c) The second reaction mixture is incubated for at least about 50 min. at about 37°C. (d) About 0.5 µM to 2.0 µM β-galactosidase EA and an excess of substrate, usually about 2 mM to about 10 mM CPRG, is combined with the second reaction mixture to form a color-forming reaction mixture. (e) The color-forming reaction mixture is incubated at about 37°C for at least about 1 min. (f) The optical density of the color-forming reaction mixture is determined. (g) The color-forming reaction mixture is incubated at about 37°C for about an additional at least about 1 min. (h) The optical density of the color-forming reaction mixture is determined. (i) The difference in optical density measurements of steps (f) and (h) are determined and compared to the difference in optical density for a sample of known concentration. A graph of optical density versus concentration using control samples can be used to determine the concentration of the samples.

A method for determining the amount of T3 present in a serum sample uses monoclonal antibodies. The method comprises the following steps in the following order: (a) About 5 nM to 50 nM β-galactosidase ED-T3 conjugate is incubated in a reaction buffer with monoclonal mouse anti-T3 antibody for about 10 to 30 min. at about 37°C to form a first reaction mixture. The anti-T3 antibody is present in a sufficient amount in comparison to ED-analyte conjugate which will substantially achieve shutdown. (b) About one volume of the first reaction mixture is combined with about four volumes of the sample to form a second reaction mixture. (c) The second reaction mixture is incubated for at least about 15 min. at about 37°C. (d) About 1.5 X 10⁻⁷ M to 7.5 X 10⁻⁷ M β-galactosidase EA and excess substrate, usually about 2 mM to about 6 mM CPRG, are combined with the second reaction mixture to form a color-forming reaction mixture. (e) The color-forming reaction mixture is incubated at about 37°C for at least about 1 min. (f) The optical density of the color-forming reaction mixture is determined. (g) The color-forming reaction mixture is incubated at about 37°C for at least about 1 min. (h) The optical density of the color-forming reaction mixture is determined. (i) The difference in optical density measurements of steps (f) and (h) is determined and compared to the difference in optical density for a sample of known T3 concentration.

A kit containing reagents facilitating the present invention is also contemplated. The kit comprises β-galactosidase ED-analyte conjugate/anti-analyte antibody complex in a first container and in a second container an enzyme acceptor. The enzyme substrate can be included with either the ED-analyte conjugate/anti-antibody complex or the EA, conveniently the complex. The ED-analyte conjugate/anti-analyte antibody complex can be formulated in dry form as a unit dosage tablet containing a quantity of reagents suitable for assay of one sample. The EA may also be provided in tablet form. Each tablet will be free from analyte but may contain enzyme substrate. The complex-containing tablet will usually be free from EA and the EA-containing tablet free from ED-analyte conjugate and anti-analyte antibody. Each tablet may additionally contain buffer. The tablets may be formulated with conventional additives such as stabilizers, drying agents, excipients and the like. The kit may additionally contain high and intermediate analyte calibrator controls. Buffer and enzyme substrate may also be included in the kit when not provided in the complex-and EA-containing reagents.

### EXPERIMENTAL

### Example 1

Example 1 is an exemplary determination of the relative concentrations of ED-analyte and anti-analyte antibodies necessary to achieve shutdown. It has been determined that some enzyme donor-analyte conjugate reacts with enzyme acceptor more rapidly than it reacts with anti-analyte antibody when all are present in a reaction mixture. Therefore, the enzyme donor analyte conjugate was incubated with various dilutions of anti-analyte antibody prior to the addition of enzyme acceptor.

In an exemplary assay, the relative concentrations of ED-digoxin and anti-digoxin antibodies were determined. The buffer used was: 100 mM NaPO₄ and 150 mM KPO₄; 10 mM EGTA (ethylene-bis-oxyethylenenitrilo tetraacetic acid); 20 mM NaN₃; 0.05% Tween 20; 0.05 mM dithiothreitol; 2 mM Mg(OAc)₂ · 4 H₂O; 6.25 mg/ml ONPG; pH 7.0. Solutions were stored on ice until used.

ED-analyte concentration was 3.8 nM. A polyclonal rabbit anti-digoxin antibody was used at serial twofold dilutions beginning with a dilution of 1:1000. The enzyme donor and antibody were incubated for 30 min. at 37°C prior to addition of EA. The incubations were run in duplicate with one of the series of dilutions additionally containing goat anti-rabbit serum (GARS) at a dilution of 1:50. Optical density of the solution was determined at 300 sec. and 420 sec. to determine the enzyme catalyzed reaction rate using ONPG as the enzyme substrate.

**TABLE 1**

| DIGOXIN ASSAY ANTIBODY TITRATION RATES | | |
|---|---|---|
| Antibody Dilution | mA/120 sec -GARS | mA/120 sec +GARS |
| 1:1000 | 180 | 35 |
| 1:2000 | 190 | 40 |
| 1:4000 | 212 | 60 |
| 1:8000 | 242 | 138 |
| 1:16000 | 280 | 222 |
| 1:32000 | 295 | 265 |
| 1:64000 | 302 | 280 |

The shutdown reaction occurred between a dilution of 1:4000 and 1:16,000 of antibody. At each antibody dilution, the presence of GARS produced a lower enzyme activity level.

### Example 2

Example 2 was a determination of whether there was a difference in enzymatic activity between 0 ng and 100 ng digoxin samples. ED-digoxin and anti-digoxin antibodies were incubated for 30 min. at 37°C. Antibody dilutions of 1:2000, 1:4000, 1:8000, 1:16,000 and 1:32,000 were used with 3.8 nM ED-digoxin. Duplicate samples were assayed in the following manner. 0 and 100 ng-containing digoxin standards were added to each complex. A first group was incubated for 50 min. at 37°C prior to addition of EA and substrate. In the second set of samples, the standards were added together with EA and substrate, and thus there was no second incubation.

**TABLE 2**

| DIGOXIN ASSAY RATES | | | |
|---|---|---|---|
| ng/mL Calibrator | Ab Dilution | 50 min. Incubation | 0 min. Incubation |
| 0 | 1:2000 | 27 | 29 |
| 100 | | 85 | 32 |
| 0 | 1:4000 | 41 | 46 |
| 100 | | 102 | 47 |
| 0 | 1:8000 | 91 | 120 |
| 100 | | 136 | 111 |
| 0 | 1:16000 | 131 | 116 |
| 100 | | 164 | 115 |
| 0 | 1:32000 | 151 | 218 |
| 100 | | 181 | 211 |

It was determined that in the absence of a second incubation there was no significant difference in the enzyme activity rate of the 0 and 100 ng digoxin samples when the optical density was measured at 5 and 7 min. following addition of EA.

### Example 3

Example 3 determined the effect of the time period of the second incubation on the change in the enzyme-catalyzed reaction rate. Incubation times between 0 and 100 min. at 10 min. intervals were determined. The change in optical density was determined in the period of from 5 to 7 min. after addition of EA and enzyme substrate. The percentage of inhibition of the reaction rate was calculated as follows. The change in optical density of a 0 ng digoxin sample (0% analyte control) as subtracted from the change in optical density for a 100 ng digoxin standard sample (100% analyte control). That number was divided by the change in optical density for a 100 ng sample to determine the percentage of inhibition. The percentage of inhibition for 0, 10, 20, 30, 40, 50 and 60 min. was 0, 24, 37, 45, 51, 59, and 60, respectively. Therefore, the first 30 min. were more important than the final 30 min., but the reaction of ED-analyte/anti-analyte complex with analyte had not reached completion within the first hour.

### Example 4

Example 4 determined an optimum ED-analyte concentration using a fixed anti-analyte antibody dilution. In this experiment, the ED-analyte conjugate concentration was varied from 2.3 nM to 6.8 nM ED-digoxin concentration using an antibody dilution of 1:4000. The second incubation (ED-analyte conjugate/anti-analyte antibody complex incubated with known concentrations of analyte) was for 60 min. After addition of EA, the optical density of the reaction mixture was read at 300 and 420 sec. to determine the enzyme catalyzed reaction rate. The percentage of modulation is the difference between any two calibrators; i.e., the net difference divided by the rate of the higher calibrator. The percentage of modulation between the 4 ng and 0 ng calibrators were 23.5, 26.0, 35.0, 36.2, 27.9, 23.0 and 19.1 for 2.3, 3.0, 3.8, 4.6, 5.3, 6.1 and 6.8 nM ED concentration, respectively. Thus, for a 1:4000 dilution of the antibody used, the modulation peaks at an ED concentration between 3.8 nM and 4.6 nM.

### Example 5

This example determined the feasibility of performing the assay using a serum sample as 50% of the reaction mixture with a more concentrated ED-digoxin/antibody complex. 0, 4 and 100 ng digoxin controls were used with 3.8 nM and 4.6 nM ED conjugate wherein the serum volume comprised 20 and 50% of the sample, respectively. The second incubation was 30 min. More concentrated ED-digoxin/antibody complex with 50% serum samples worked and gave somewhat better results than reaction mixtures with less than 50% serum samples.

### Example 6

This example compared the use of ONPG as a substrate with the use of chlorophenol red galactoside (CPRG) as the enzyme substrate. The ONPG concentration was 1.33 mg/ml and the CPRG concentration was 0.8 mg/ml in the system. Using CPRG enhanced the optical density of each value (signal). The optical density values were approximately 7 times as large using CPRG. However, the difference in the percentage of inhibition and modulation was not significant. That is, although each value was greater, the ratio of the values was the same.

### Example 7

An exemplary digoxin assay combining the results of the previous examples was performed as follows. The concentration of each of the reagents is listed below. The concentration of the reagent which is added to the reaction mixture is given as is the reagent's concentration in the final reaction mixture (system value).

ED-Analyte = 34 nM reagent = 3.4 nm system
Antibody = 1:500 reagent = 1:5000 system
GARS = 1:6.25 reagent = 1:62.5 system
Serum Sample = 50% of second reaction mixture = 10% system
Enzyme Acceptor = 1.2 mM reagent = 0.69 mM system
CPRG = 4 mg/ml reagent = 0.8 mg/ml system
The immune complex was incubated for 30 min. at 37°C. Sample was added and incubated for an additional 10 min. The optical density was read at 90 and 210 sec. from addition of EA to determine the rate. The percentage of inhibition using a 100 ng and 0 ng calibrator was 44.3%, while the percentage of modulation using the 4 ng and 0 ng calibrators was 30.2%.

### Example 8

An assay for T3 in serum using a mouse monoclonal antibody (Lot No. A7696) was performed as follows. The concentration of reagents was
ED-Analyte = 5.8 X 10⁻⁸ M reagent = 2 X 10⁻⁹ M system
Antibody = 1:800 reagent = 1:20,000 system
EA = 15 u/test = 450 nM system
The enzyme donor conjugate and antibody were incubated for 45 min. at 37°C in a water bath. 10 µl of ED-analyte/antibody complex was added to 40 µl of sample and incubated for 15 min. at 37°C. 200 µl of EA and substrate were added and incubated at 37°C. The optical density of the solution was read at 60 and 180 sec. following addition of EA. Modulation between 0 and 8 ng/ml was found to peak between 1:800 and 1:1000 antibody dilutions at a value of 47 to 48%. The concentration of EA was varied between 39 u/test to 1.5 u/test. The inhibition and modulation improved between 39 to 12 u/test with reduced EA. Below 12 u/test there was very little difference in percent inhibition and percent modulation with changing EA concentration. The addition of goat anti-mouse serum to the original solution was found to make no significant difference in modulation and a minimial difference in inhibition.

The present invention eliminates incubation steps, permits mixing of most of the assay reagents, and requires less technican time. Additionally, the reagents can be formulated as a unit dose for ease of manipulation and convenient packaging. It has been shown experimentally that the assay has a positive response where more analyte gives more signal. The method is ideally suited for field use as it is performed using fewer reagents and steps.

## Claims

1. A method for determining the amount of analyte present in a sample comprising:
(a) admixing a β-galactosidase enzyme donor-analyte (ED-analyte) conjugate complexable with anti-analyte antibody to form a complex, a said anti-analyte antibody, a β-galactosidase enzyme acceptor (EA), enzyme substrate and said sample to form a reaction mixture; and
(b) determining the rate of enzyme-catalyzed reaction in said reaction mixture as indicative of the amount of analyte present in said sample, characterised in that substantially all ED-analyte conjugate and anti-analyte antibody present in the reaction mixture are admixed in the form of said complex.

2. A method for determining the amount of analyte present in a sample comprising:
(a) admixing in a reaction buffer a β-galactosidase ED-analyte conjugate complexable with anti-analyte antibody to form a complex, a said anti-analyte antibody and said sample to form a first reaction mixture; and
(b) combining a β-galactosidase enzyme acceptor (EA) with said first reaction mixture for a time sufficient for EA to react with ED to form β-galactosidase to form a second reaction mixture,
wherein enzyme substrate is added to said first or second reaction mixture; and
(c) determining the rate of enzyme-catalyzed reaction in said second reaction mixture as indicative of the amount of analyte present in said sample, characterized in that substantially all ED-analyte conjugate and anti-analyte antibody present in the reaction mixture are admixed in the form of said complex and in that the first reaction mixture is incubated for a time sufficient for said complex to react with analyte present in said sample.

3. A method according to claim 2 wherein the ED-analyte/anti-analyte antibody complex is formed by incubating ED-analyte conjugate in an aqueous medium with sufficient anti-analyte antibody to substantially achieve the lowest enzyme activity possible in the presence of ED-analyte conjugate, anti-analyte antibody, EA and substrate for a time sufficient to substantially complete reaction.

4. A method according to claim 2 or claim 3 further characterized in that said ED-analyte/anti-analyte antibody complex is combined with a second antibody specific for the Fc portion of said anti-analyte antibody to form an ED-analyte/anti-analyte antibody/second antibody complex prior to step (a).

5. A method according to claim 2 for determining the amount of analyte present in a serum sample wherein the steps (a), (b) and (c) are characterized in that they comprise:
(a) combining in a reaction buffer a β-galactosidase ED-analyte conjugate/anti-analyte antibody complex with said sample for a time sufficient for anti-analyte antibody to react with analyte present in said sample to form a first reaction mixture;
(b) combining a β-galactosidase EA with said reaction mixture in the presence of enzyme substrate for a time sufficient to form a β-galactosidase enzyme to form a second reaction mixture; and
(c) determining the change in light absorbence or emission by the product of the enzyme-catalyzed reaction of said second reaction mixture as indicative of the amount of analyte present in said sample.

6. A method according to claim 5 wherein said determining step (c) comprises determining the difference in optical density of said second reaction mixture following incubation at about 37°C at least about 1 min. after the combining of step (b) and a second time at least about 1 min. later.

7. A method according to claim 5 for determining the amount of digoxin present in a serum sample which includes incubating in a reaction buffer about 30 mM to 40 mM β-galactosidase ED-digoxin conjugate with polyclonal anti-digoxin antibody and second antibody specific for said anti-digoxin antibody for at least about 30 min. at about 37°C wherein said anti-digoxin antibody and said ED-analyte conjugate are present in amounts which substantially achieve shutdown which is the lowest enzyme activity possible in the presence of ED-analyte conjugate, anti-analyte antibody, EA and substrate, to form the ED-analyte conjugate/anti-analyte antibody complex; wherein steps (a) and (b) comprise
(a) combining an equal volume of said sample with said complex to form a reaction mixture,and incubating said reaction mixture for about 10 to about 30min at about 37°C; and
(b) combining about 0.5 mM to 2.0 mM β-galactosidase EA and about 2 mM to about 10 mM chlorophenol red galactoside (CPRG) with said reaction mixture to form a color-forming reaction mixture;
and step (c) comprises performing the steps of;
(e) incubating said color-forming reaction mixture at about 37°C for at least about 1 min.;
(f) determining the optical density of said color-forming reaction mixture;
(g) incubating said color-forming reaction mixture at about 37°C for at least about 1 min.;
(h) determining the optical density of said color-forming reaction mixture;
(i) determining the difference in optical density measurements of steps (f) and (h); and
(j) comparing said difference to the difference in optical density for a sample of known concentration.

8. A method according to claim 5 for determining the amount of T-3 present in a serum sample which includes incubating in a reaction buffer about 5nM to 50 nM β-galactosidase ED-T3 conjugate with monoclonal anti-T3 antibody for about 45 min. at about 37°C wherein said anti-T3 antibody and said ED-analyte conjugate are present in amounts which substantially achieve shutdown which is the lowest enzyme activity possible in the presence of ED-analyte conjugate, anti-analyte antibody, EA and substrate, to form the ED-analyte conjugate/anti-analyte antibody complex, wherein steps (a) and (b) comprise;
(a) combining about one volume of said complex with about four volumes of said sample to form a reaction mixture, and incubating said reaction mixture for at least about 15 min. at about 37°C, and
(b) combining β-galactosidase EA and CPRG with said reaction mixture to form a color forming reaction mixture having about 1.5 X 10⁻⁷ M to 7.5 X 10⁻⁷ M β-galactosidase EA and from about 2 mM to about 6mM CPRG; and
step (c) comprises performing the steps of;
(e) incubating said color-forming reaction mixture at about 37°C for at least about 1 min.;
(f) determining the optical density of said color-forming reaction mixture;
(g) incubating said color-forming reaction mixture at about 37°C for at least about 1 min.;
(h) determining the optical density of said color-forming reaction mixture;
(i) determining the difference in optical density measurements of steps (f) and (h); and
(j) comparing said difference to the difference in optical density for a sample of known concentration.

9. A reagent tablet comprising β-galactosidase ED-analyte conjugate/anti-analyte antibody complex.

10. A kit comprising in a first container β-galactosidase ED-analyte conjugate/anti-analyte antibody complex substantially free from analyte and β-galactosidase EA in a second container substantially free from ED-analyte conjugate, anti-analyte antibody, enzyme substrate and analyte.

## Patentansprüche

1. Verfahren zum Bestimmen der in einer Probe vorhandenen Analytmenge, umfassend:
(a) das Mischen eines β-Galaktosidaseenzym-Donoranalyt(ED-Analyt)-Konjugats, das mit Antianalytantikörper komplexierbar ist, um einen Komplex zu bilden, eines genannten Antianalytantikörpers, eines β-Galaktosidaseenzym-Akzeptors (EA), Enzymsubstrats und der genannten Probe, um eine Reaktionsmischung zu bilden; und
(b) das Bestimmen der Rate der enzymkatalysierten Umsetzung in der genannten Reaktionsmischung als Indikator für die in der genannten Probe vorhandene Analytmenge, dadurch gekennzeichnet, daß im wesentlichen alles des in der Reaktionsmischung vorhandenen ED-Analytkonjugats und Antianalytantikörpers in Form des genannten Komplexes gemischt werden.

2. Verfahren zum Bestimmen der in einer Probe vorhandenen Analytmenge, umfassend:
(a) das Mischen eines β-Galaktosidase-ED-Analyt-Konjugats, das mit Antianalytantikörper komplexierbar ist, um einen Komplex zu bilden, eines genannten Antianalytantikörpers und der genannten Probe in einem Reaktionspuffer, um eine erste Reaktionsmischung zu bilden; und
(b) das Kombinieren eines β-Galaktosidaseenzym-Akzeptors (EA) mit der genannten ersten Reaktionsmischung für einen Zeitraum, der ausreicht, um EA mit ED umzusetzen, sodaß β-Galaktosidase gebildet wird, um eine zweite Reaktionsmischung zu bilden,
worin Enzymsubstrat der genannten ersten oder zweiten Reaktionsmischung hinzugefügt wird; und
(c) das Bestimmen der Rate der enzymkatalysierten Umsetzung in der genannten zweiten Reaktionsmischung als Indikator für die in der genannten Probe vorhandene Analytmenge, dadurch gekennzeichnet, daß im wesentlichen alles des in der Reaktionsmischung vorhandenen ED-Analytkonjugats und Antianalytantikörpers in Form des genannten Komplexes gemischt werden und dadurch, daß die erste Reaktionsmischung für einen Zeitraum inkubiert wird, der ausreicht, damit der genannte Komplex mit in der genannten Probe vorhandenem Analyten umgesetzt wird.

3. Verfahren nach Anspruch 2, worin der ED-Analyt/Antianalytantikörper-Komplex gebildet wird, indem ED-Analytkonjugat in einem wässerigen Medium mit ausreichend Antianalytantikörper inkubiert wird, um im wesentlichen die geringstmögliche Enzymaktivität in Gegenwart von ED-Analytkonjugat, Antianalytantikörper, EA und Substrat für einen Zeitraum, der für im wesentlichen vollständige Reaktion ausreicht, zu erzielen.

4. Verfahren nach Anspruch 2 oder 3, das weiters dadurch gekennzeichnet ist, daß der genannte ED-Analyt/Antianalytantikörper-Komplex mit einem zweiten Antikörper kombiniert wird, der für den Fc-Anteil des genannten Antianalytantikörpers spezifisch ist, um vor Schritt (a) einen Komplex aus ED-Analyt, Antianalytantikörper und zweitem Antikörper zu bilden.

5. Verfahren nach Anspruch 2 zum Bestimmen der in einer Serumprobe vorhandenen Analytmenge, worin die Schritte (a), (b) und (c) dadurch gekennzeichnet sind, daß sie umfassen:
(a) das Kombinieren eines β-Galaktosidase-ED-Analytkonjugat/Antianalytantikörper-Komplexes mit der genannten Probe in einem Reaktionspuffer für einen Zeitraum, der zur Umsetzung von Antianalytantikörper mit in der genannten Probe vorhandenem Analyten ausreicht, um eine erste Reaktionsmischung zu bilden;
(b) das Kombinieren eines β-Galaktosidase-EAs mit der genannten Reaktionsmischung in Gegenwart von Enzymsubstrat für einen ausreichenden Zeitraum, um ein β-Galaktosidaseenzym zu bilden, um eine zweite Reaktionsmischung zu bilden; und
(c) das Bestimmen der Veränderung der Lichtabsorbanz bzw. -extinktion oder -emission durch das Produkt der enzymkatalysierten Umsetzung der genannten zweiten Reaktionsmischung als Indikator für die in der genannten Probe vorhandene Analytmenge.

6. Verfahren nach Anspruch 5, worin der genannte Bestimmungsschritt (c) das Bestimmen der Differenz der optischen Dichte der genannten zweiten Reaktionsmischung nach Inkubation bei etwa 37°C für zumindest etwa 1 Minute nach dem Kombinieren von Schritt (b) und ein zweitesmal zumindest etwa 1 Minute später umfaßt.

7. Verfahren nach Anspruch 5 zum Bestimmen der in einer Serumprobe vorhandenen Digoxinmenge, welches das Inkubieren von etwa 30 mM bis 40 mM β-Galaktosidase-ED-Digoxin-Konjugat mit polyklonalem Antidigoxinantikörper und einem zweiten für den genannten Antidigoxinantikörper spezifischen Antikörper in einem Reaktionspuffer für zumindest etwa 30 min bei etwa 37°C umfaßt, worin der genannte Antidigoxinantikörper und das genannte ED-Analytkonjugat in Mengen vorhanden sind, die im wesentlichen Shutdown, der die geringstmögliche Enzymaktivität in Gegenwart von ED-Analytkonjugat, Antianalytantikörper, EA und Substrat darstellt, um den ED-Analytkonjugat/Antianalytantikörper-Komplex zu bilden; worin die Schritte (a) und (b) umfassen
(a) das Kombinieren eines gleichen Volumens der genannten Probe mit dem genannten Komplex, um eine Reaktionsmischung zu bilden, und das Inkubieren der genannten Reaktionsmischung für etwa 10 bis etwa 30 min bei etwa 37°C; und
(b) das Kombinieren von etwa 0,5 mM bis 2,0 mM β-Galaktosidase-EA und etwa 2 mM bis etwa 10 mM Chlorphenolrotgalaktosid (CPRG) mit der genannten Reaktionsmischung, um eine farbbildende Reaktionsmischung zu bilden;
und Schritt (c) das Durchführen folgender Schritte umfaßt:
(e) das Inkubieren der genannten farbbildenden Reaktionsmischung bei etwa 37°C für zumindest etwa 1 min;
(f) das Bestimmen der optischen Dichte der genannten farbbildenden Reaktionsmischung;
(g) das Inkubieren der genannten farbbildenden Reaktionsmischung bei etwa 37°C für zumindest etwa 1 min;
(h) das Bestimmen der optischen Dichte der genannten farbbildenden Reaktionsmischung;
(i) das Bestimmen der Differenz in den Messungen der optischen Dichte der Schritte (f) und (h); und
(j) das Vergleichen der genannten Differenz mit der Differenz der optischen Dichte einer Probe mit bekannter Konzentration.

8. Verfahren nach Anspruch 5 zum Bestimmen der in einer Serumprobe vorhandenen Menge an T-3, welches das Inkubieren von etwa 5 nM bis 50 nM β-Galaktosidase-ED-T3-Konjugat mit monoklonalem Anti-T3-Antikörper für etwa 45 min in einem Reaktionspuffer bei etwa 37°C umfaßt, worin der genannte Anti-T3-Antikörper und das genannte ED-Analytkonjugat in Mengen vorhanden sind, die im wesentlichen Shutdown erzielen, der die geringstmögliche Enzymaktivität in Gegenwart von ED-Analykonjugat, Antianalytantikörper, EA und Substrat darstellt, um den ED-Analytkonjugat/Antianalytantikörper-Komplex zu bilden, worin die Schritte (a) und (b) umfassen:
(a) das Kombinieren von etwa 1 Volumen des genannten Komplexes mit etwa 4 Volumina der genannten Probe, um eine Reaktionsmischung zu bilden, und das Inkubieren der genannten Reaktionsmischung für zumindest etwa 15 min bei etwa 37°C, und
(b) das Kombinieren von β-Galaktosidase-EA und CPRG mit der genannten Reaktionsmischung, um eine farbbildende Reaktionsmischung mit etwa 1,5 X 10⁻⁷ M bis 7,5 X 10⁻⁷ M β-Galaktosidase-EA und von etwa 2 mM bis etwa 6 mM CPRG zu bilden; und Schritt (c) die Durchführung folgender Schritte umfaßt:
(e) das Inkubieren der genannten farbbildenden Reaktionsmischung bei etwa 37°C für zumindest etwa 1 min;
(f) das Bestimmen der optischen Dichte der genannten farbbildenden Reaktionsmischung;
(g) das Inkubieren der genannten farbbildenden Reaktionsmischung bei etwa 37°C für zumindest etwa 1 min;
(h) das Bestimmen der optischen Dichte der genannten farbbildenden Reaktionsmischung;
(i) das Bestimmen der Differenz der Meßergebnisse für die optische Dichte der Schritte (f) und (h); und
(j) das Vergleichen der genannten Differenz mit der Differenz der optischen Dichte einer Probe mit bekannter Konzentration.

9. Reagenstablette, die β-Galaktosidase-ED-Analytkonjugat/Antianalytantikörper-Komplex enthält.

10. Set, das in einem ersten Behälter β-Galaktosidase-ED-Analytkonjugat/Antianalytantikörper-Komplex, der im wesentlichen frei von Analyt ist, und in einem zweiten Behälter β-Galaktosidase-EA umfaßt, der im wesentlichen frei von ED-Analytkonjugat, Antianalytantikörper, Enzymsubstrat und Analyt ist.

## Revendications

1. Méthode pour déterminer la quantité d'un analyte présent dans un échantillon consistant à :
(a) mélanger un conjugué donneur d'enzyme de β-galactosidase-analyte (ED-analyte) pouvant être complexé avec un anticorps anti-analyte pour former un complexe, ledit anticorps anti-analyte, un accepteur d'enzyme β-galactosidase (EA), un substrat d'enzyme et ledit échantillon pour former un mélange réactionnel; et
(b) déterminer l'allure de la réaction catalysée par l'enzyme dans ledit mélange réactionnel comme indiquant la quantité de l'analyte présent dans ledit échantillon, caractérisée en ce que substantiellement la totalité du conjugué ED-analyte et de l'anticorps anti-analyte se trouvant dans le mélange réactionnel sont mélangés sous la forme dudit complexe.

2. Méthode pour déterminer la quantité d'un analyte présent dans un échantillon consistant à :
(a) mélanger, dans un tampon de réaction, un conjugué ED de β-galactosidase-analyte pouvant être complexé avec un anticorps anti-analyte pour former un complexe, ledit anticorps anti-analyte et ledit échantillon pour former un premier mélange réactionnel; et
(b) combiner un accepteur d'enzyme de β-galactosidase (EA) avec ledit premier mélange réactionnel pendant un temps suffisant pour que EA réagisse avec ED pour former une β-galactosidase afin de former un second mélange réactionnel,
où le susbstrat d'enzyme est ajouté audit premier ou second mélange réactionnel; et
(c) déterminer l'allure de la réaction catalysée par l'enzyme dans ledit second mélange réactionnel comme indiquant la quantité de l'analyte présent dans ledit échantillon, caractérisée en ce que sensiblement la totalité du conjugué ED-analyte et de l'anticorps anti-analyte présents dans le mélange réactionnel sont mélangés sous la forme dudit complexe et en ce qu'on soumet à incubation le premier mélange réactionnel pendant un temps suffisant pour que ledit complexe réagisse avec l'analyte présent dans ledit échantillon.

3. Méthode selon la revendication 2 où le complexe ED-analyte/anticorps anti-analyte est formé par incubation du conjugué ED-analyte dans un milieu aqueux avec suffisamment de l'anticorps anti-analyte pour obtenir sensiblement la plus faible activité enzymatique possible en présence du conjugué ED-analyte, de l'anticorps anti-analyte, de EA et du substrat, pendant un temps suffisant pour terminer sensiblement la réaction.

4. Méthode selon la revendication 2 ou la revendication 3, caractérisée en ce que ledit complexe ED-analyte/anticorps anti-analyte est combiné à un second anticorps spécifique de la portion Fc dudit anticorps anti-analyte pour former un complexe ED-analyte/anticorps anti-analyte/second anticorps avant l'étape (a).

5. Méthode selon la revendication 2, pour déterminer la quantité d'un analyte présent dans un échantillon de sérum où les étapes (a), (b) et (c) sont caractérisées en ce qu'elles consistent à :
(a) combiner, dans un tampon de réaction, un complexe conjugué ED de β-galactosidase-analyte/anticorps anti-analyte avec ledit échantillon pendant un temps suffisant pour que l'anticorps anti-analyte réagisse avec l'analyte présent dans ledit échantillon pour former un premier mélange réactionnel;
(b) combiner un EA de β-galactosidase avec ledit mélange réactionnel en présence du substrat d'enzyme pendant un temps suffisant pour former une enzyme de β-galactosidase afin de former un second mélange réactionnel; et
(c) déterminer le changement de l'absorbance de la lumière ou de l'émission par le produit de la réaction catalysée par l'enzyme dudit second mélange réactionnel comme indiquant la quantité de l'analyte présent dans ledit échantillon.

6. Méthode selon la revendication 5 où ladite étape consistant à déterminer (c) consiste à déterminer la différence de densité optique dudit second mélange réactionnel suivant l'incubation aux environs de 37°C au moins environ 1 minute après la combinaison de l'étape (b) et une seconde fois au moins environ 1 minute plus tard.

7. Méthode selon la revendication 5, pour déterminer la quantité de digoxine présente dans un échantillon de sérum, qui consiste à soumettre à incubation, dans un tampon réactionnel, un conjugué ED de β-galactosidase-digoxine 30 mM à 40 mM avec un anticorps polyclonal anti-digoxine et un second anticorps spécifique dudit anticorps anti-digoxine pendant au moins environ 30 minutes aux environs de 37°C, où ledit anticorps anti-digoxine et ledit conjugué ED-analyte sont présents en quantités qui permettent d'obtenir sensiblement l'arrêt qui est la plus faible activité enzymatique possible en présence du conjugué ED-analyte, de l'anticorps anti-analyte, de EA et du substrat afin de former le complexe conjugué ED-analyte/anticorps anti-analyte; où les étapes (a) et (b) consistent à :
(a) combiner un volume égal dudit échantillon avec ledit complexe pour former un mélange réactionnel et soumettre ledit mélange réactionnel à incubation pendant environ 10 à environ 30 mn aux environs de 37°C; et
(b) combiner EA de la β-galactosidase 0,5 mM à 2,0 mM et du galactosiderouge de chlorophénol (CPRG) 2 mM à environ 10 mM avec ledit mélange réactionnel pour former un mélange réactionnel formant une couleur; et l'étape (c) consiste à accomplir les étapes de :
(e) soumettre ledit mélange réactionnel formant une couleur à incubation aux environs de 37°C pendant au moins environ 1 mn;
(f) déterminer la densité optique dudit mélange réactionnel formant une couleur;
(g) soumettre ledit mélange réactionnel formant une couleur à incubation aux environs de 37°C pendant au moins environ 1 mn;
(h) déterminer la densité optique dudit mélange réactionnel formant une couleur;
(i) déterminer la différence des mesures de densité optique des étapes (f) et (h); et
(j) comparer ladite différence à la différence de densité optique pour un échantillon d'une concentration connue.

8. Méthode selon la revendication 5 pour déterminer la quantité de T-3 que l'on trouve dans un échantillon de sérum, qui consiste à soumettre à incubation, dans un tampon réactionnel, le conjugé ED de β-galactosidase-T3 5nM à 50 nM avec un anti-corps monoclonal anti-T3 pendant environ 45 mn, aux environs de 37°C, où ledit anticorps anti-T3 et ledit conjugué ED-analyte sont présents en quantités qui permettent d'obtenir sensiblement l'arrêt qui est la plus basse activité enzymatique possible en présence du conjugué ED-analyte, de l'anticorps anti-analyte, de EA et du substrat pour former le complexe conjugué de ED-analyte/anticorps anti-analyte où les étapes (a) et (b) consistent à :
(a) combiner environ 1 volume dudit complexe avec environ quatre volumes dudit échantillon pour former un mélange réactionnel et soumettre ledit mélange réactionnel à incubation pendant au moins environ 15 mn. aux environs de 37°C et
(b) combiner EA de β-galactosidase et CPRG avec ledit mélange réactionnel pour former un mélange réactionnel formant une couleur ayant environ 1,5 x 10⁻⁷M à 7,5 x 10⁻⁷M de EA de β-galactosidase et environ 2 mM à environ 6 mM de CPRG; et l'étape (c) consiste à accomplir les étapes de :
(e) soumettre ledit mélange réactionnel formant une couleur à incubation aux environs de 37°C pendant au moins environ 1 mn;
(f) déterminer la densité optique dudit mélange réactionnel formant une couleur;
(g) soumettre ledit mélange réactionnel formant une couleur à incubation aux environs de 37°C pendant au moins 1 mn.;
(h) déterminer la densité optique dudit mélange réactionnel de formation d'une couleur;
(i) déterminer la différence des mesures de densité optique des étapes (f) et (h); et
(j) comparer ladite différence à la différence de densité optique d'un échantillon d'une concentration connue.

9. Comprimé réactif comprenant un complexe conjugué ED de β-galactosidase-analyte/anticorps anti-analyte.

10. Trousse comprenant, dans un premier conteneur, un complexe conjugué de ED de β-galactosidase-analyte/anticorps anti-analyte sensiblement sans analyte et ED de β-galactosidase dans un second conteneur sensiblement sans conjugué ED-analyte, anticorps anti-analyte substrat enzymatique ni analyte.
